Europäisches Patentamt

European Patent Office

Office européen des brevets

(11) Publication number: **0 318 195 B1**

(19)
(12)

# EUROPEAN PATENT SPECIFICATION

(45) Date of publication of patent specification: **05.08.92**  (51) Int. Cl.⁵: **A61F 5/448**

(21) Application number: **88310735.1**

(22) Date of filing: **14.11.88**

(54) **Ostomy coupling having integral O-ring coupling.**

(30) Priority: **16.11.87 US 121387**

(43) Date of publication of application:
**31.05.89 Bulletin 89/22**

(45) Publication of the grant of the patent:
**05.08.92 Bulletin 92/32**

(84) Designated Contracting States:
**AT BE CH DE ES FR GB GR IT LI LU NL SE**

(56) References cited:
**WO-A-88/01156**
**FR-A- 2 548 890**

(73) Proprietor: **E.R. Squibb & Sons, Inc.**
**Lawrenceville-Princeton Road**
**Princeton, N.J. 08540-4000(US)**

(72) Inventor: **Freeman, Frank Michael**
**10 Brandon Road**
**Lewrenceville New Jersey(US)**

(74) Representative: **Cook, Anthony John et al**
**D. YOUNG & CO. 10, Staple Inn**
**London, WC1V 7RD(GB)**

## Description

The present invention relates to a coupling device. In particular, the invention provides a two-piece, O-ring coupling design which can be used to seal an ostomy pouch to a body side flange.

Ostomy couplings are used to connect body side flanges, which are typically adhesively attached to the body of a wearer, to matching couplings which are typically attached to disposable bags.

It is quite important that the seal between the body side member and the bag side member be secure, and to the extent possible, be free of leakage of gases or fluids.

PCT International Application WO88/01156, was published on 25th February 1988 subsequent to the priority date of the present application. It is hence an EPC Article 54(3) document. It discloses an ostomy appliance comprising a coupling for joining a pad or dressing to an ostomy bag including a first member of closed loop form for defining a stoma aperture therein said first member having a formation which defines two opposed walls, and a second member of closed loop form also defining a stoma aperture, the second member having a rib or projection dimensioned and positioned to fit between and resiliently engage with both of said walls when the members are connected in such a way as to make a sealing engagement therebetween. The two opposed walls each include an inwardly extending region thereby defining a narrowed opening between said walls and each of the two opposite sides of the rib or projection includes a laterally extending region disposed such that said latter regions pass between said narrowed opening when the two parts of the coupling are interconnected and engage said inwardly extending regions.

British Patent GB-A-1 583 027 (FR-A-2 548 890) discloses an ostomy coupling in which two members of the coupling (bag side member and body side member) are connected in such a way as to make sealing engagement with at least the said one wall, the rib or projection having an enlarged end portion of a width as seen in cross-section greater than the minimum width between the opposed walls.

The rib or projection is gripped between the two walls of the second member in the mutually coupled positions of the members.

In contrast to these arrangements, in the present invention, a dual O-ring is compressed to provide a resilient seal.

According to the present invention, there is provided an ostomy coupling of the type comprising a bag side member and a body side member and having at least one elastomeric member which is formed as an integral part of said body side member and serves to seal said member; and channel means formed in said bag side member, said channel means being adapted to receive and to compress a part of the body side member, whereby in use said bag side member is sealably attached to said body side member; characterised in that the elastomeric member is an O-ring which takes the form of a dual O-ring having radially inner and radially outer O-ring portions.

The invention will be better understood from the following non-limiting description of an example thereof given with reference to the accompanying drawings in which:-

Figure 1 is a perspective view illustrating the body side flange and bag side flange of an embodiment of the present invention;

Figure 2 is a side cross-sectional view of the body side flange and bag side flange shown in Figure 1;

Figure 3 is a bottom view of the bag side flange of the embodiment shown in Figure 1 taken along the lines 8-8 of Figure 2;

Figure 4 is a top view of the body side flange of Figure 1 taken along the lines 9-9 of Figure 2; and

Figure 5 is a cross-sectional view showing the assembled bag side flange and body side flange of Figure 1.

Referring to the Figures, an ostomy coupling is comprised of a bag side member 112 and a body side member 114. As is standard in the art, the bag side member is attached to an ostomy bag 16, and the body side member includes a flange 18 which is used to attach the body side member to a pad of adhesive 20 for use in attaching the body side member 114 to the body of a wearer (not shown). The body side member 114 includes an opening which overlies a similar opening which is formed in the adhesive pad 20. These openings are placed over the stomal opening of the wearer.

As will be shown, the present invention contemplates the use of a seal which includes an O-ring 124 which is made to be an integral part of the coupling.

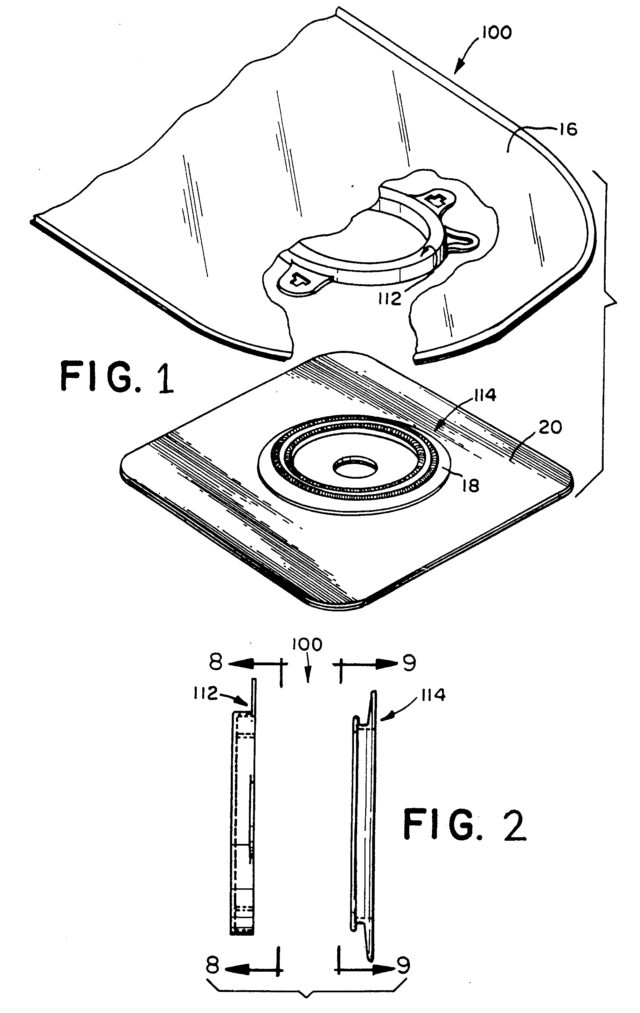

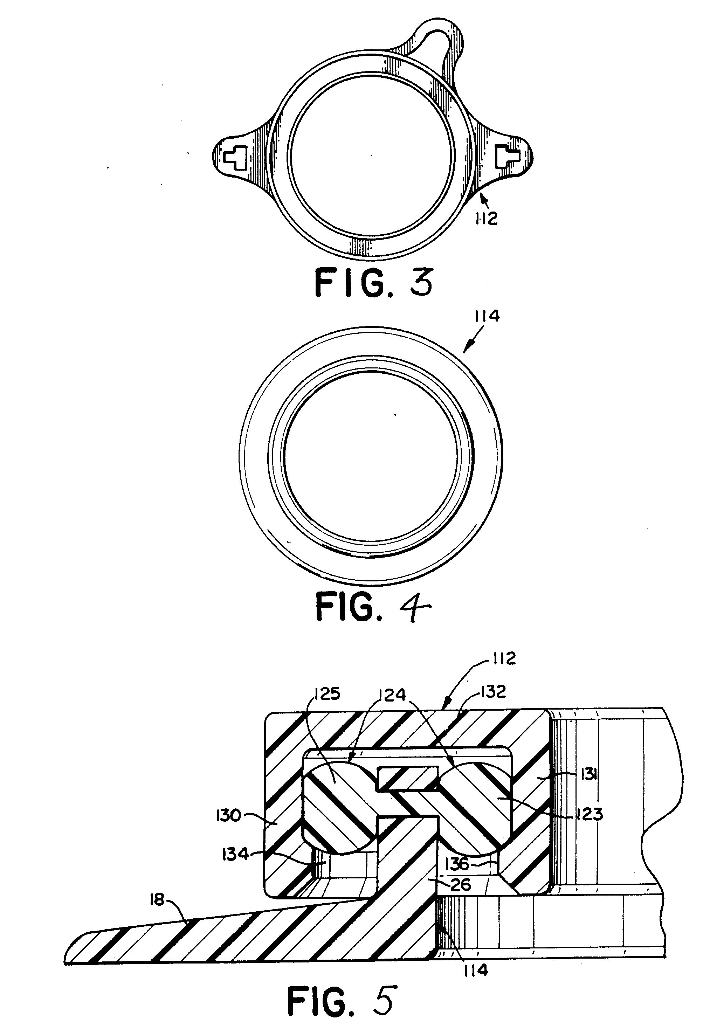

Referring specifically to Figures 1-5, an ostomy coupling 100, Figure 2, is comprised of bag side member 112 and body side member 114. The bag side member 112 is connected to ostomy bag 16, Figure 1, and the body side member 114 includes a flange 18 which is used to attach the body side member 114 to an adhesive pad 20. With reference to Figures 1-5, it is clear that the wall 26 of the body side member 18 carries a dual O-ring 124 including both an inner portion 123 and an outer portion 125 which are inside and outside respectively, the cylindrical wall 26 which is attached to the flange 18.

The bag side member 112 includes both an outer cylindrical leg 130 and an inner cylindrical leg 131, which together with the bag attachment flange 132 form a substantially U-shaped channel which fits over the dual O-ring 124 such that when the bag side member 112 is applied over the dual O-ring 124, the raised annular sections 134, 136 snap over the outer and inner portions 125, 123 of the dual O-ring 124. As will be recognised by one of ordinary skill in the art, in manufacturing the body side member 114, one may merely place the dual O-ring 124 into a mold prior to forming the flange 18 in wall 26 structure.

## Claims

1. An ostomy coupling of the type comprising a bag side member (112) and a body side member (114) and having at least one elastomeric member which is formed as an integral part of said body side member and serves to seal said member; and channel means formed in said bag side member, said channel means being adapted to receive and to compress a part of the body side member, whereby in use said bag side member is sealably attached to said body side member; characterised in that the elastomeric member is an O-ring which takes the form of a dual O-ring (124) having radially inner and radially outer O-ring portions (123, 125).

## Revendications

1. Raccord de stomie du type comprenant un élément (112) côté sac et un élément (114) côté corps et comportant au moins une pièce élastomère qui fait partie intégrante dudit élément côté corps et sert à le rendre étanche ; et une pièce à section en U formée dans ledit élément côté sac, ladite pièce à section en U étant conçue pour recevoir et comprimer une partie de l'élément côté corps, de sorte qu'à l'utilisation ledit élément côté sac est fixé de façon étanche audit élément côté corps ; caractérisé en ce que la pièce élastomère est un joint torique qui se présente sous la forme d'un joint torique double (124) ayant une partie radialement intérieure (123) et une partie radialement extérieure (125).

## Patentansprüche

1. Kupplung für einen Ostomiebeutel, die ein beutelseitiges Glied (112) und ein körperseitiges Glied (114), mindestens ein elastisches Glied, das mit dem körperseitigen Glied einstückig ist und dieses Glied abdichtet, und in dem beutelseitigen Glied ausgebildete Kanäle aufweist, die einen Teil des körperseitigen Gliedes aufnehmen und zusammenpressen können, so daß beim Gebrauch das beutelseitige Glied abdichtend am körperseitigen Glied befestigt wird, **dadurch gekennzeichnet,** daß das elastische Glied ein O-Ring ist, der die Form eines doppelten O-Ringes (124) mit radial inneren und radial äußeren O-Ringabschnitten (123, 125) annimmt.

100

16

112

# FIG. 1

114

20

18

8

100

9

112

114

8

9

# FIG. 2

4

EP 0 318 195 B1

FIG. 3

FIG. 4

FIG. 5